(19) Europäisches Patentamt
European Patent Office
Office européen des brevets

(11) Veröffentlichungsnummer: **0 030 380**
**A1**

(12) # EUROPÄISCHE PATENTANMELDUNG

(21) Anmeldenummer: **80107669.6**

(22) Anmeldetag: **05.12.80**

(51) Int. Cl.³: **C 07 D 209/38**, A 61 K 31/40

(30) Priorität: **07.12.79 GB 7942324**

(43) Veröffentlichungstag der Anmeldung: **17.06.81**
**Patentblatt 81/24**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Fothergill, Graham Alwyn, 7 Stockens Dell, Knebworth, Herts. (GB)**
Erfinder: **Osbond, John Mervyn, 4 Lodge Drive, Hatfield, Herts (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **2,3-Indoldionderivate, Verfahren zu ihrer Herstellung, Zwischenprodukte, Arzneimittel enthaltend solche 2,3-Indoldionderivate und ihre pharmazeutische Verwendung.**

(57) Die neuen Verbindungen der allgemeinen Formel

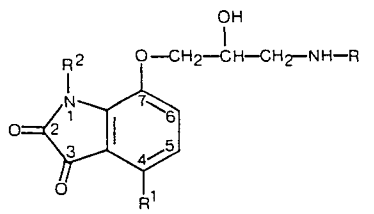

I

worin R Isopropyl oder t-Butyl, $R^1$ Wasserstoff oder niederes Alkyl und $R^2$ niederes Alkyl oder niederes Aralkyl bedeuten,
und ihre pharmazeutisch annehmbaren Säureadditionssalze werden hergestellt, indem man eine Verbindung der Formel

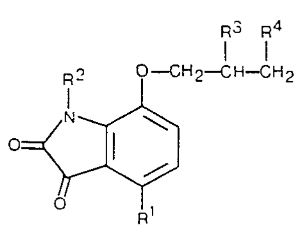

II

worin $R^3$ Hydroxy und $R^4$ Chlor oder Brom bedeuten oder $R^3$ und $R^4$ zusammen ein Sauerstoffatom bedeuten, und $R^1$ und $R^2$ obige Bedeutung besitzen,
mit einem Amin der Formel

$$H_2N\text{-}R \qquad III$$

worin R obige Bedeutung besitzt,
umsetzt, erwünschtenfalls ein erhaltenes Racemat in die beiden Antipoden auftrennt und erwünschtenfalls eine erhaltene Verbindung der Formel I in ein pharmazeutisch annehmbares Säureadditionssalz überführt. Die Verbindungen der Formel I und ihre oben erwähnten Salze sind nützlich als β-adrenergische Blocker und Antihypertensiva.

EP 0 030 380 A1

9. Dez. 1980
0030380

F.Hoffmann-La Roche & Co.Aktiengesellschaft, Basel/Schweiz

RAN 4020/28

# 2,3-Indoldionderivate, Verfahren zu ihrer Herstellung, Zwischenprodukte, Arzneimittel enthaltend solche 2,3-Indoldionderivate und ihre pharmazeutische Verwendung

Die vorliegende Erfindung betrifft 2,3-Indoldionderivate. Im speziellen betrifft sie 2,3-Indoldionderivate, ein Verfahren für ihre Herstellung, pharmazeutische Präparate enthaltend diese Derivate und die Verwendung dieser Derivate.

Die erfindungsgemässen 2,3-Indoldionderivate sind Verbindungen der allgemeinen Formel

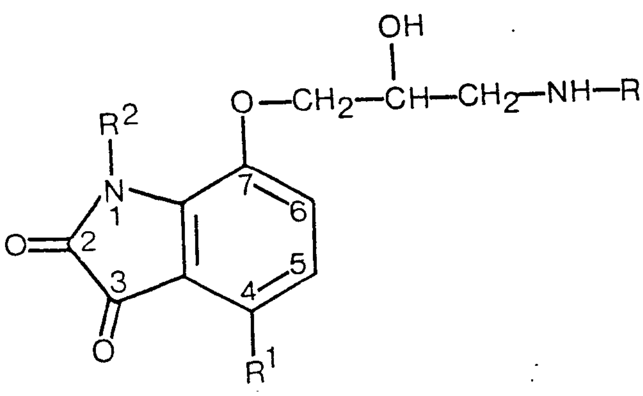

I

worin R Isopropyl oder t-Butyl, $R^1$ Wasserstoff oder niederes Alkyl und $R^2$ niederes Alkyl oder niederes Aralkyl bedeuten,
und pharmazeutisch annehmbare Säureadditionssalze davon.

Nt/14.11.1980

Der Ausdruck "niederes Alkyl", wie er in der vorliegenden Beschreibung verwendet wird, bedeutet geradkettige oder verzweigte Alkylgruppen, welche vorzugsweise 1 bis 6 Kohlenstoffatome enthalten (z.B. Methyl, Aethyl, Propyl, Isopropyl, Butyl, Isobutyl, t-Butyl, Pentyl, Hexyl, usw.). Der Ausdruck "niederes Aralkyl", wie er in der vorliegenden Beschreibung verwendet wird, bedeutet eine niedere Alkylgruppe, wie sie oben definiert ist, worin ein Wasserstoffatom durch eine Arylgruppe substituiert ist. Beispiele solcher niederer Aralkylgruppen sind Benzyl, β-Phenäthyl, usw.

Eine bevorzugte Klasse von 2,3-Indoldionderivaten, gemäss vorliegender Erfindung, umfasst Verbindungen der Formel I, worin $R^1$ Wasserstoff und $R^2$ niederes Alkyl bedeuten, und pharmazeutisch annehmbare Säureadditionssalze davon.

Bevorzugte Verbindungen der Formel I sind:

7-(3-t-Butylamino-2-hydroxypropoxy)-1-methyl-2,3-indoldion,

7-(3-t-Butylamino-2-hydroxypropoxy)-1,4-dimethyl-2,3-indoldion,

7-(2-Hydroxy-3-isopropylaminopropoxy)-1,4-dimethyl-2,3-indoldion und

1-Benzyl-7-(3-t-butylamino-2-hydroxypropoxy)-4-methyl-2,3-indoldion.

Die 2,3-Indoldionderivate der Formel I und ihre pharmazeutisch annehmbaren Säureadditionssalze können erfindungsgemäss dadurch hergestellt werden, dass man

a) eine Verbindung der allgemeinen Formel

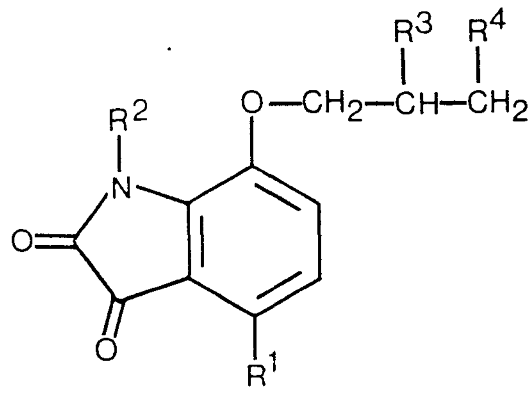

II

worin $R^3$ Hydroxy und $R^4$ Chlor oder Brom bedeuten, oder $R^3$ und $R^4$ zusammen ein Sauerstoffatom bedeuten, und $R^1$ und $R^2$ obige Bedeutung besitzen,
mit einem Amin der allgemeinen Formel

$$H_2N-R \qquad III$$

worin R obige Bedeutung besitzt,
umsetzt,

b) erwünschtenfalls ein erhaltenes Racemat in die beiden Antipoden auftrennt, und/oder

c) erwünschtenfalls eine erhaltene Verbindung der Formel I in ein pharmazeutisch annehmbares Säureadditionssalz überführt.

Die Reaktion einer Verbindung der Formel II mit einem Amin der Formel III (d.h. Isopropylamin oder t-Butylamin) kann in an sich bekannter Weise durchgeführt werden. Die Reaktion kann in der Gegenwart eines inerten organischen Lösungsmittels erfolgen. Verwendet man ein inertes organisches Lösungsmittel, so kommen niedere Alkanole, wie Methanol oder Aethanol, in Frage. Andererseits kann man auch einen Ueberschuss an Amin der Formel III einsetzen, sodass dieses gleichzeitig als Lösungsmittel dient. Die Reaktion wird vorteilhafterweise in einem Temperaturbereich von etwa 0°C bis Raumtemperatur, vorzugsweise bei Raumtemperatur und Atmosphärendruck, durchgeführt.

Es sei besonders hervorgehoben, dass die Verbindungen der Formel I ein asymmetrisch substituiertes Kohlenstoffatom besitzen, und somit in racemischen oder optisch aktiven Formen auftreten können. Die vorliegende Erfindung umfasst sowohl die Racemate als auch die optisch aktiven Formen. Ein Racemat kann erwünschtenfalls nach an sich bekannten Methoden in die optischen Isomeren aufgetrennt werden; beispielsweise durch fraktionierte Kristallisation von Salzen, welche mit optisch aktiven Säuren erhalten wurden.

Die Verbindungen der Formel I können durch Behandeln mit einer pharmazeutisch annehmbaren anorganischen Säure (z.B. Salzsäure, Bromwasserstoff, Schwefelsäure, Phosphorsäure, usw.) oder mit einer pharmazeutisch annehmbaren organischen Säure (z.B. Essigsäure, Weinsäure, Zitronensäure, Fumarsäure, Maleinsäure, Apfelsäure, Methansulfonsäure, p-Toluolsulfonsäure, usw.) in pharmazeutisch annehmbare Säureadditionssalze übergeführt werden.

Die als Ausgangsstoffe verwendeten Verbindungen der Formel II können beispielsweise gemäss dem nachfolgenden Reaktionsschema, worin $R^1$ und $R^2$ obige Bedeutung besitzen und $R^5$ niederes Alkyl, insbesondere Methyl bedeutet, hergestellt werden:

<u>Formelschema</u>

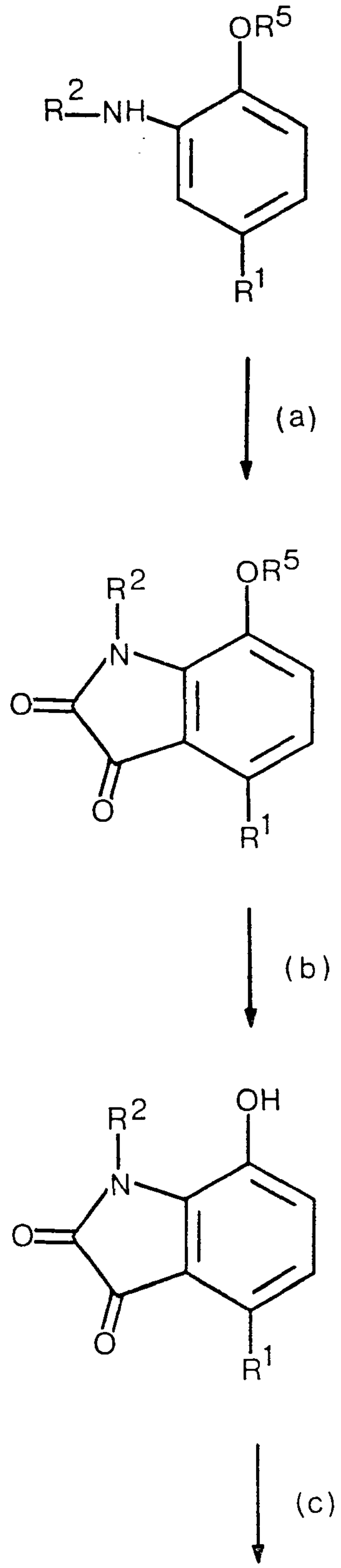


IV

V

VI

Gemäss Schritt a) des obigen Formelschemas wird eine Verbindung der Formel IV, eine an sich bekannte Verbindung oder eine Verbindung, welche in an sich bekannter Weise leicht hergestellt werden kann, in eine Verbindung der Formel V umgewandelt. Diese Umwandlung wird durchgeführt, indem man eine Verbindung der Formel IV mit Oxalylchlorid in einem inerten organischen Lösungsmittel, vorzugsweise in einem aromatischen Kohlenwasserstoff, wie Benzol, in einem Temperaturbereich von etwa 50°C bis 80°C, umsetzt und das erhaltene Reaktionsprodukt in einem hochsiedenden inerten organischen Lösungsmittel, vorzugsweise in einem nitrierten aromatischen Kohlenwasserstoff, wie Nitrobenzol, Nitrotoluol, etc., auf eine Temperatur oberhalb etwa 160°C erhitzt.

Gemäss Schritt b) des obigen Formelschemas wird die niedere Alkoxygruppe $-OR^5$ in einer Verbindung der Formel V in an sich bekannter Weise in die Hydroxygruppe übergeführt; beispielsweise mit Bortribromid bei niederen Temperaturen (z.B. bei etwa -70°C), Pyridinhydrochlorid bei hohen Temperaturen (z.B. bei etwa 200°C), etc. Es sei festgehalten, dass wenn $R^2$ niederes Aralkyl bedeutet, eine Methode verwendet werden muss, welche diese Gruppe nicht beeinträchtigt.

Gemäss Schritt c) des obigen Formelschemas wird ein Phenol der Formel VI mit einem Epichlorhydrin oder Epibromhydrin, vorzugsweise Epichlorhydrin, in an sich bekannter Weise umgesetzt. In einer bevorzugten Ausführungsform wird die Reaktion in der Gegenwart eines basischen Anionenaustauscherharzes, wie Amberlyst A-26, unter Verwendung eines Ueberschusses an Epichlorhydrin oder Epibromhydrin und bei erhöhter Temperatur (z.B. etwa 60°C) durchgeführt. In einer weiteren Ausführungsform kann die Reaktion in Gegenwart eines Alkalimetallhydroxyds (z.B. Natriumhydroxyd oder Kaliumhydroxyd) und zweckmässigerweise in Gegenwart eines mit Wasser mischbaren organischen Lösungsmittels (z.B. eines niederen Alkanols, wie Methanol oder Aethanol)

in einem Temperaturbereich von etwa 0°C bis etwa Raumtemperatur, vorzugsweise bei Raumtemperatur, durchgeführt werden.

Das aus obiger Reaktion erhaltene Produkt ist eine Mischung aus einem Epoxyd der allgemeinen Formel

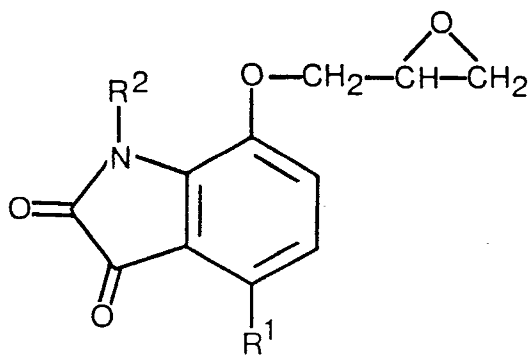

IIa

worin $R^1$ und $R^2$ obige Bedeutung besitzen,
und dem entsprechenden Chlorhydrin oder Bromhydrin der allgemeinen Formel

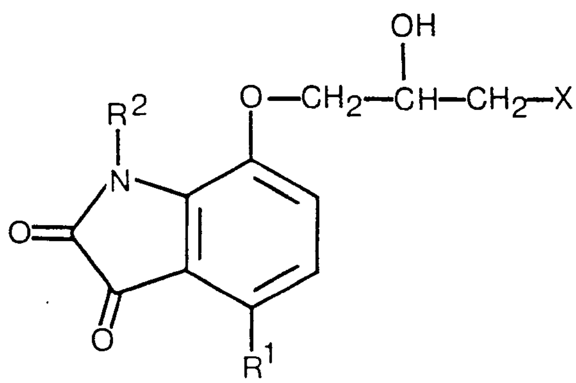

IIb

worin $R^1$ und $R^2$ obige Bedeutung besitzen und X Chlor oder Brom bedeutet,
wobei das Chlorhydrin oder Bromhydrin der Formel IIb normalerweise nur in geringen Mengen vorkommt.

Die erfindungsgemässen 2,3-Indoldionderivate (d.h. Verbindungen der Formel I und ihre pharmazeutisch annehmbaren Säureadditionssalze) besitzen β-adrenergische Blockereigenschaften. Sie können demtentsprechend bei der Bekämpfun

bzw. Verhütung von Herzkrankheiten, wie beispielsweise Angina pectoris und Herzrhytmusstörungen, verwendet werden. Sie können auch als antihypertensive Wirkstoffe verwendet werden.

Die β-adrenergischen Blockereigenschaften der erfindungsgemässen 2,3-Indoldionderivate können mittels Standardtestverfahren nachgewiesen werden. In einem solchen Testverfahren wird die β-adrenergische Blockereigenschaft an Ratten dadurch gezeigt, dass man diejenige Dosierung ($ED_{50}$) der zu testenden Verbindung in µg/kg i.v. ermittelt, welche notwendig ist, um die Isoprenalin-induzierte Tachycardie um 50% zu reduzieren. In diesem Test wurden für 7-(3-t-Butylamino-2-hydroxypropoxy)-1-methyl-2,3-indoldion-hydrochlorid, 7-(3-t-Butylamino-2-hydroxypropoxy)-1,4-dimethyl-2,3-indoldion-hydrochlorid bzw. 7- (2-Hydroxy-3-isopropylamino-propoxy)-1,4-dimethyl-2,3-indoldion-hydrochlorid $ED_{50}$-Werte von 20, 26 bzw. 49 µg/kg i.v. gefunden, währenddem für Propanolol, einen bekannten und vielfach verwendeten β-adrenergischen Blocker, ein $ED_{50}$-Wert von 143 µg/kg i.v. gefunden wurde.

Die Verbindungen der Formel I und ihre pharmazeutisch annehmbaren Säureadditionssalze können als Arzneimittel in Form von pharmazeutischen Präparaten, welche diese in Kombination mit einem verträglichen pharmazeutischen Trägermaterial enthalten, verwendet werden. Dieses Trägermaterial kann ein inertes organisches oder anorganisches Trägermaterial sein, das für die enterale (z.B. orale) oder parenterale Verabreichung geeignet ist. Als Beispiele solcher Trägermaterialien seien Wasser, Gelatine, Lactose, Stärke, Magnesiumstearat, Talk, Gummi arabicum, Polyalkylenglykole, pflanzliche Oele, Vaselin und dergleichen genannt. Die pharmazeutische Präparate können in einer festen Dosierungsform (z.B. als Tabletten, Dragées, Suppositorien oder Kapseln) oder in flüssiger Dosierungsform (z.B. als Lösungen, Suspensionen oder Emulsionen) vorliegen. Die pharmazeutischen Präparate können herkömmlichen pharmazeutischen Operationen, wie Sterilisation, unterworfen werden. Weiterhin können pharmazeutische Präparate herkömmliche Hilfsmittel, wie Konservierungsmittel, Stabilisierungsmittel, Emulgiermittel, Aromastoffe, Netzmittel, Salze, um den osmotischen Druck zu verändern, Puffer, und dergleichen, enthalten.

Eine Verbindung der Formel I oder ein pharmazeutisch annehmbares Säureadditionssalz davon kann Erwachsenen in einer täglichen Dosierung von etwa 1 mg/kg bis 10 mg/kg in einer einzigen Dosis oder in mehreren Dosen verabreicht werden. Es sei festgehalten, dass die obigen Dosierungsangaben lediglich als Beispiele dienen, und dass die Dosierungen nach oben oder nach unten, in Abhängigkeit von Faktoren, wie die zu verabreichende Verbindung oder das zu verabreichende Salz, die Art der Verabreichung und die Bedürfnisse des Patienten, wie sie vom behandelnden Arzt festgestellt werden, variieren können.

Die folgenden Beispiele erläutern das erfindungsgemässe Verfahren.

Beispiel 1

Eine Suspension von 5 g 1-Methyl-7-hydroxy-2,3-indoldion und 5,5 g Amberlyst-Harz A-26 in 50 ml Epichlorhydrin wird unter Rühren während 6 Stunden auf 60°C erwärmt. Anschliessend wird vom Harz abfiltriert und zur Trockene eingedampft. Das erhaltene dunkelrote Oel wird an Kieselgel unter Eluieren mit Chloroform chromatographiert. Fraktionen, welche das gewünschte Produkt enthalten, werden vereinigt und eingedampft. Man erhält 3,82 g eines roten Oeles, das langsam aus Hexan kristallisiert. Dieses Produkt wird ohne weitere Reinigung in 100 ml Aethanol suspendiert und mit 16 ml t-Butylamin versetzt. Man rührt während 2 Tagen bei 20°C, dampft die Mischung anschliessend ein, und behandelt den Rückstand mit äthanolischer Salzsäure. Nach Kristallisieren aus Aethanol erhält man 1,25 g 7-(3-t-Butylamino-2-hydroxypropoxy)-1-methyl-2,3-indoldion-hydrochlorid als orange Kristalle vom Schmelzpunkt 245-247°C.

Die folgenden Hydrochloride werden in analoger Weise hergestellt:

7-(3-t-Butylamino-2-hydroxypropoxy)-1,4-dimethyl-2,3-indoldion-hydrochlorid vom Schmelzpunkt 258-261°C und

7-(2-Hydroxy-3-isopropylaminopropoxy)-1,4-dimethyl-2,3-indoldion-hydrochlorid vom Schmelzpunkt 227-228°C.

Das im ersten Absatz dieses Beispiels als Ausgangsstoff verwendete 1-Methyl-7-hydroxy-2,3-indoldion kann beispielsweise wie folgt hergestellt werden:

a) Eine Lösung von 25,3 ml (0.22 M) o-Anisidin in 300 ml Pyridin wird über einen Zeitraum von 0,5 Stunden bei einer Temperatur unterhalb 30°C mit 47 ml (0,33 M) Trifluoressigsäureanhydrid behandelt. Man lässt die Mischung während 3 Stunden bei 20°C stehen und giesst sie anschliessend auf 2 l Wasser. Der erhaltene Festkörper wird abfiltriert und aus Wasser umkristallisiert; man erhält 29,5 g N-Trifluoracetyl-o-anisidin als rosafarbenen Festkörper vom Schmelzpunkt 42-43°C.

b) 55,25 g (0,25 Mol) N-Trifluoracetyl-o-anisidin in 200 ml Aceton werden mit 62 ml (1.0 Mol) Methyljodid und 65 g (1.0 Mol) Kaliumhydroxyd während 0,5 Stunden unter Rückfluss zum Sieden erhitzt. Nach Eindampfen der Mischung zur Trockne versetzt man mit Wasser und erhitzt das erhaltene Gemisch während 2 Stunden unter Rückfluss, um die Hydrolyse zu vervollständigen.Die Base wird mit Aether ausgeschüttelt und mit Wasser gewaschen. Man erhält 34,2 g N-Methyl-o-anisidin, das beim Stehenlassen kristallisiert; Schmelzpunkt 28-30°C.

c) Eine gerührte Lösung von 38 ml (0,42 Mol) Oxalylchlorid in 400 ml Benzol wird bei 50°C tropfenweise mit 52 g (0,38 Mol) N-Methyl-o-anisidin in 600 ml trockenem Benzol versetzt. Nach beendeter Zugabe wird die Lösung während 5 Stunden unter Rückfluss zum Sieden erhitzt und anschliessend zur Trockne eingedampft. Der Rückstand wird in 300 ml Nitrobenzol aufgenommen und unter Stickstoff während 7 Stunden auf 160°C erhitzt. Die rote Lösung wird zur Trockne eingedampft; der Rückstand wird aus Aethanol kristallisiert. Man erhält 29,01 g 1-Methyl-7-methoxy-2,3-indoldion vom Schmelzpunkt 173-176°C.

d) 17 g 1-Methyl-7-methoxy-2,3-indoldion in 340 ml Methylenchlorid werden bei -70°C tropfenweise mit 25,5 ml Bortribromid in 255 ml Methylenchlorid versetzt. Nach beendeter Zugabe wird die Mischung während 1 Stunde bei 0°C und anschliessend während 2 Stunden bei 20°C gerührt. Ueberschüssiges Bortribromid wird bei -70°C mit Methanol zerstört. Die Mischung wird anschliessend zur Trockne eingedampft. Der feste Rückstand wird mit 170 ml Wasser behandelt, abfiltriert und mit Wasser gewaschen. Man erhält 14,86 g 1-Methyl-7-hydroxy-2,3-indoldion vom Schmelzpunkt 154-166°C. Dieses Material wird ohne weitere Reinigung direkt weiterverarbeitet

Beispiel 2

Eine Mischung aus 2,53 g 1-Benzyl-4-methyl-7-hydroxy-2,3-indoldion, 25 ml Epichlorhydrin und 2,53 g Amberlyst-Harz A-26 wird während 2 Tagen bei 20°C gerührt. Nach Entfernen des Harzes wird die Mischung zur Trockne eingedampft. Eine Mischung aus 17,62 g des erhaltenen roten Festkörpers und 88,8 ml t-Butylamin in 200 ml Methanol wird während 4,5 Stunden unter Rückfluss zum Sieden erhitzt. Man versetzt mit Wasser und dampft das Methanol ab. Das Produkt wird mit Methylenchlorid extrahiert und an Kieselgel unter Eluieren mit Chloroform und schliesslich mit Methanol/Chloroform (20%) chromatographiert. Die Fraktionen, welche das benötigte Produkt enthalten, werden vereinigt. Das Produkt wird in Isopropanol in das Oxalsäuresalz übergeführt (5,8 g Base benötigen 1,42 g Oxalsäure). Nach Umkristallisieren aus Isopropanol/Diäthyläther erhält man 5,28 g des Oxalsäuresalzes von 1-Benzyl-7-(3-t-butylamino-2-hydroxypropoxy)-4-methyl-2,3-indoldion das oberhalb 220°C schmilzt. Durch Behandeln dieses Salzes mit Natronlauge in Methylenchlorid und Kristallisieren des erhaltenen Produktes aus Cyclohexan erhält man 2,9 g der freien Base vom Schmelzpunkt 125-128°C.

Das als Ausgangsmaterial verwendete 1-Benzyl-4-methyl-7-hydroxy-2,3-indoldion kann beispielsweise wie folgt hergestellt werden:

a) Eine Mischung aus 10 g (0,073 Mol) 2-Methoxy-5-methylanilin und 16,3 ml (0,16 Mol) Benzaldehyd in 80 ml Methanol wird während 6 Stunden unter Rückfluss zum Sieden erhitzt. Man versetzt die abgekühlte Lösung unter Rühren mit 2 g Natriumborhydrid und lässt die Mischung anschliessend während 8 Stunden stehen. Man versetzt mit Wasser, entfernt das Methanol, nimmt das Produkt in Essigester auf und wäscht es mit 2N Salzsäure und Wasser. Nach Entfernen des Essigesters und Kristallisieren des Rückstandes aus Isopropanol erhält man 6,48 g N-Benzyl-2-methoxy-5-methylanilin vom

Schmelzpunkt 50-57,5°C.

b) 25,15 g (0,198 Mol) Oxalylchlorid in 250 ml Benzol werden unter Rühren bei 60°C über einen Zeitraum von 1 Stunde mit 41,13 g (0,18 Mol) N-Benzyl-2-methoxy-5-methylanilin versetzt. Man erhitzt während 3 Stunden unter Rückfluss zum Sieden, entfernt das Lösungsmittel und versetzt mit Nitrotoluol. Die erhaltene Mischung wird unter Stickstoff während 5 Stunden auf 160°C erhitzt. Der rote Festkörper, den man nach Entfernen des Nitrotoluols erhält, wird aus Isopropanol kristallisiert. Man erhält 24,99 g 1-Benzyl-4-methyl-7-methoxy-2,3-indoldion vom Schmelzpunkt 149-152°C.

c) 15,46 g 1-Benzyl-4-methyl-7-methoxy-2,3-indoldion und 74 g Pyridinhydrochlorid werden während 2,5 Stunden auf 200°C erhitzt. Die Mischung wird anschliessend zwischen Methylenchlorid und wässrigem Dimethylsulphoxid verteilt. Die organische Phase wird abgetrennt und eingedampft. Man erhält 3,75 g rohes 1-Benzyl-4-methyl-7-hydroxy-2,3-indoldion als roten Festkörper vom Schmelzpunkt 255-262°C.

Die folgenden Beispiele betreffen typische pharmazeutische Präparate, enthaltend die erfindungsgemässen 2,3-Indoldionderivate:

## Beispiel A

Tabletten, welche die folgenden Bestandteile enthalten, können in herkömmlicher Weise hergestellt werden:

| | Pro Tablette |
|---|---|
| 2,3-Indoldionderivat | 25 mg |
| Lactose | 103 mg |
| Stärke | 61 mg |
| Magnesiumstearat | 11 mg |
| Totalgewicht | 200 mg |

## Beispiel B

Kapseln, enthaltend die folgenden Bestandteile, können in herkömmlicher Weise hergestellt werden:

| | Pro Kapsel |
|---|---|
| 2,3-Indoldionderivat | 25 mg |
| Lactose | 106 mg |
| Stärke | 20 mg |
| Talk | 9 mg |
| Totalgewicht | 160 mg |

## Patentansprüche

1. 2,3-Indoldionderivate der allgemeinen Formel

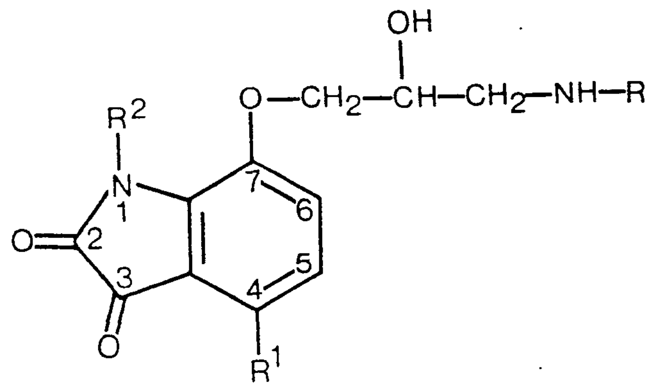

I

worin R Isopropyl oder t-Butyl, $R^1$ Wasserstoff oder niederes Alkyl und $R^2$ niederes Alkyl oder niederes Aralkyl bedeuten,
und pharmazeutisch annehmbare Säureadditionssalze davon.

2. Verbindungen gemäss Anspruch 1, worin $R^1$ Wasserstoff und $R^2$ niederes Alkyl bedeutet, und pharmazeutisch annehmbare Säureadditionssalze davon.

3. 7-(3-t-Butylamino-2-hydroxypropoxy)-1-methyl-2,3-indoldion.

4. 7-(3-t-Butylamino-2-hydroxypropoxy)-1,4-dimethyl-2,3-indoldion.

5. 7-(2-Hydroxy-3-isopropylaminopropoxy)-1,4-dimethyl-2,3-indoldion.

6. 1-Benzyl-7-(3-t-butylamino-2-hydroxypropoxy)-4-methyl-2,3-indoldion.

7. Verbindungen der allgemeinen Formel

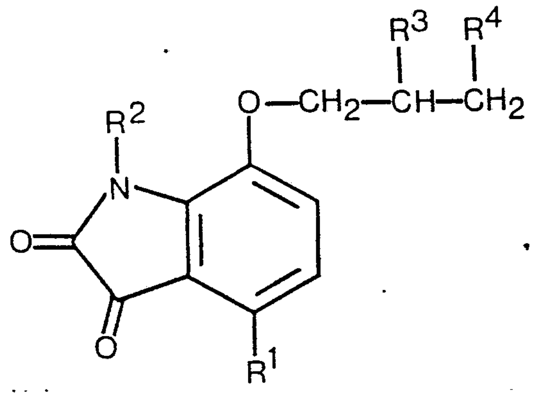

II

worin $R^1$ Wasserstoff oder niederes Alkyl, $R^2$ niederes Alkyl oder niederes Aralkyl, $R^3$ Hydroxy und $R^4$ Chlor oder Brom bedeuten oder $R^3$ und $R^4$ zusammen ein Sauerstoffatom bedeuten.

8. Verbindungen der allgemeinen Formel

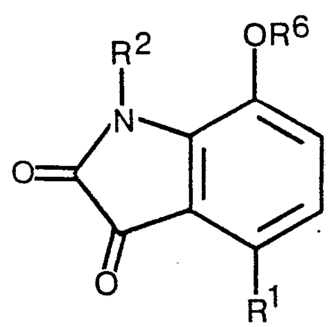

worin $R^1$ Wasserstoff oder niederes Alkyl, $R^2$ niederes Alkyl oder niederes Aralkyl und $R^6$ Wasserstoff oder niederes Alkyl bedeuten, mit der Massgabe, dass $R^2$ und/oder $R^3$ nicht Methyl bedeuten, wenn $R^1$ Wasserstoff bedeutet.

9. 2,3-Indoldionderivate gemäss einem der Ansprüche 1 bis 6 oder pharmazeutisch annehmbare Säureadditionssalze davon als pharmazeutische Wirkstoffe.

10. 2,3-Indoldionderivate gemäss einem der Ansprüche 1 bis 6 oder pharmazeutisch annehmbare Säureadditionssalze davon als β-adrenergische Blocker oder Antihypertensiva.

11. Verfahren zur Herstellung von 2,3-Indoldionderivaten der in Anspruch 1 definierten Formel I und von pharmazeutisch annehmbaren Säureadditionssalzen davon, dadurch gekennzeichnet, dass man

a) eine Verbindung der allgemeinen Formel

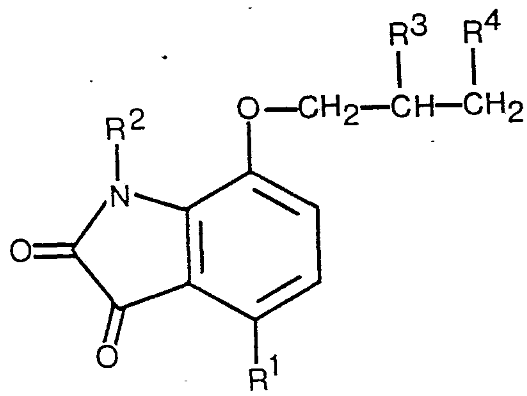

II

worin $R^3$ Hydroxy und $R^4$ Chlor oder Brom bedeuten, oder $R^3$ und $R^4$ zusammen ein Sauerstoffatom bedeuten, und $R^1$ und $R^2$ die in Anspruch 1 angegebene Bedeutung besitzen,

mit einem Amin der allgemeinen Formel

$H_2N-R$ III

worin R die in Anspruch 1 angegebene Bedeutung besitzt,

umsetzt,

b) erwünschtenfalls ein erhaltenes Racemat in die beiden Antipoden auftrennt, und/oder

c) erwünschtenfalls eine erhaltene Verbindung der Formel I in ein pharmazeutisch annehmbares Säureadditionssalz überführt.

12. Arzneimittel, enthaltend ein 2,3-Indoldionderivat gemäss einem der Ansprüche 1 bis 6 oder ein pharmazeutisch annehmbares Säureadditionssalz davon.

13. ß-Blocker oder Antihypertensivum, enthaltend ein 2,3-Indoldionderivat gemäss einem der Ansprüche 1 bis 6 oder ein pharmazeutisch annehmbares Säureadditionssalz davon.

BAD ORIGINAL

14. Verwendung von 2,3-Indoldionderivaten gemäss einem der Ansprüche 1 bis 6 oder von pharmazeutisch annehmbaren Säureadditionssalzen davon bei der Bekämpfung bzw. Verhütung von Krankheiten.

15. Verwendung von 2,3-Indoldionderivaten gemäss einem der Ansprüche 1 bis 6 oder von pharmazeutisch annehmbaren Säureadditionssalzen davon bei der Bekämpfung bzw. Verhütung von Angina pectoris, Herzrhytmusstörungen oder Hypertensionen.

***

Patentansprüche für Oesterreich

1. Verfahren zur Herstellung von 2,3-Indoldionderivaten der allgemeinen Formel

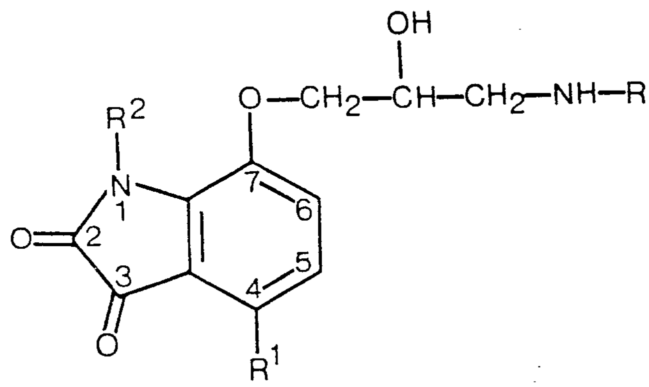

I

worin R Isopropyl oder t-Butyl, $R^1$ Wasserstoff oder niederes Alkyl und $R^2$ niederes Alkyl oder niederes Aralkyl bedeuten,

und von pharmazeutisch annehmbaren Säureadditionssalzen davon, dadurch gekennzeichnet, dass man

a) eine Verbindung der allgemeinen Formel

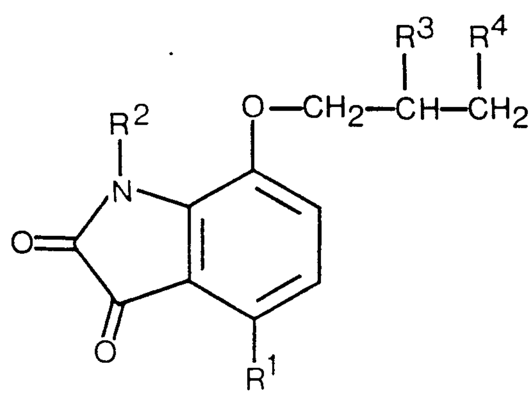

II

worin $R^3$ Hydroxy und $R^4$ Chlor oder Brom bedeuten, oder $R^3$ und $R^4$ zusammen ein Sauerstoffatom bedeuten, und $R^1$ und $R^2$ obige Bedeutung besitzen,

BAD ORIGINAL

mit einem Amin der allgemeinen Formel

$$H_2N-R \qquad III$$

worin R obige Bedeutung besitzt, umsetzt,

b) erwünschtenfalls ein erhaltenes Racemat in die beiden Antipoden auftrennt, und/oder

c) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass man Verbindungen der in Anspruch 1 definierten allgemeinen Formel I, worin $R^1$ Wasserstoff und $R^2$ niederes Alkyl bedeutet, und pharmazeutisch annehmbare Säureadditionssalze davon herstellt.

3. Verfahren gemäss Anspruch 1, dadurch gekennzeichnet, dass man 7-(3-t-Butylamino-2-hydroxypropoxy)-1-methyl-2,3-indoldion herstellt.

4. Verfahren gemäss Anspruch 1, dadurch gekennzeichnet, dass man 7-(3-t-Butylamino-2-hydroxypropoxy)-1,4-dimethyl-2,3-indoldion herstellt.

5. Verfahren gemäss Anspruch 1, dadurch gekennzeichnet, dass man 7-(2-Hydroxy-3-isopropylaminopropoxy)-1,4-dimethyl-2,3-indoldion herstellt.

6. Verfahren gemäss anspruch 1, dadurch gekennzeichnet, dass man 1-Benzyl-7-(3-t-butylamino-2-hydroxypropoxy)-4-methyl-2,3-indoldion herstellt.

BAD ORIGINAL

0030380

Nummer der Anmeldung

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

EP 80107669.6

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | ULLMANNS ENCYKLOPÄDIE DER TECHNISCHEN CHEMIE, Band 12, 4.Aufl. (1976) Verlag Chemie, Weinheim, New York + Seiten 638, 639 + -- | 1,9,10 12,13 | C 07 D 209/38<br>A 61 K 31/40 |
| | AT - B - 266 112 (SANDOZ) + Gesamt + ---- | 1,2, 9-13 | |
| | | | RECHERCHIERTE SACHGEBIETE (Int. Cl.³) |
| | | | C 07 D 209/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-13

Unvollständig recherchierte Patentansprüche:

Nicht recherchierte Patentansprüche: 14 und 15

Grund für die Beschränkung der Recherche: (Verfahren zur therapeutischen Behandlung des menschlichen oder tierischen Körpers, Art.52 (4) EPÜ)

**KATEGORIE DER GENANNTEN DOKUMENTE**

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

| Recherchenort | Abschlußdatum der Recherche | Prüfer |
|---|---|---|
| WIEN | 02-03-1981 | BRUS |